Europäisches Patentamt

European Patent Office

Office européen des brevets

(11) Publication number: **0 494 664 A1**

# EUROPEAN PATENT APPLICATION

(21) Application number: **92100257.2**

(22) Date of filing: **09.01.92**

(51) Int. Cl.5: **C12N 15/12**, A61K 37/02, C07K 3/08, C07K 13/00

(30) Priority: **09.01.91 GB 9100381**

(43) Date of publication of application: **15.07.92 Bulletin 92/29**

(84) Designated Contracting States: **PT**

(71) Applicant: **FARMITALIA CARLO ERBA S.r.L.**
Via Carlo Imbonati 24
I-20159 Milano(IT)

(72) Inventor: **Cauet, Gilles**
8, rue du Maréchal Leclerc
F-67370 Griesheim sur Souffel(FR)
Inventor: **Caccia, Paolo**
Via S. Margherita No. 89
I-21042 Caronno Pertusella (Varese)(IT)
Inventor: **Nitti, Giampaolo**
Via Monte Cervino, 28
I-20052 Monza (Milan)(IT)
Inventor: **Bertolero, Federico**
Via Pallavicino 22
I-20100 Milan(IT)

(74) Representative: **Kolb, Helga, Dr. Dipl.-Chem. et al**
Hoffmann, Eitle & Partner Patentanwälte
Arabellastrasse 4
W-8000 München 81(DE)

(54) **Human bFGF derivatives, their analogs and process for their production.**

(57) Recombinant human basic fibroblast growth factors or functionally equivalent analogs thereof are provided in which at least one of the four cysteine amino acid residues is derivatized. Usually the two cysteines corresponding to those present in positions 69 and 87 of the 146 amino acid form of the natural human basic fibroblast growth factor are derivatized by moieties such as for example $-CH_2COOH$ or $-CH_2CONH_2$.

Such derivatized recombinant human basic fibroblast growth factors may be used for the healing of wounds, ulcers or burns; regeneration of damaged neural tissues; aiding tissue transplant or healing bone graft; or revascularization of ischaemic tissues.

The present invention relates to derivatives of human basic fibroblast growth factor (bFGF) and its functionally equivalent analogues, as well as to a process for their production.

Human bFGF belongs to a family of structurally related proteins which have been isolated from normal and tumour tissues.

Besides basic FGF, the family includes acidic FGF and the proto-oncogene products int-2, hst/ks, FGF-5, FGF-6 and FGF-7/kGF.

Basic FGF, the first of the factors to be isolated and biologically characterized, has been more thoroughly investigated than its relatives.

It is widely distributed in tissues (central and peripheral nervous systems, retina, kidney and myocardium) and tumours (breast and bladder carcinoma, hepatoma and glioma).

bFGF was originally extracted from the pituitary gland as a factor exhibiting a mitogenic activity on fibroblasts and endothelial cells (Gospodarowicz; Nature, 249, 123, 1974). The discovery that bFGF has a high affinity for heparin and related glycosaminoglycans rapidly led to the isolation, by means of a heparin affinity chromatography, of several mitogens which are very similar or identical to basic FGF.

These mitogens have been given names based either on the tissue of origin or on target cell specificity, and include, among others, cartilage-derived growth factor (CDGF) (Sullivan R., Klagsbrum M.; J. Biol. Chem. 260, 2399-2403; 1985), heparin-binding growth factor β (HBGF-β) (Lobb R.R., et al.; Biochem. Biophys. Res.Comm. 131, 586-592, 1985), eye-derived growth factor-1 (EDGF-1) (Courty J. et al. Biochimie 67, 265-269, 1985) and astroglial growth factor-2 (AGF-2) (Pettman B., et al; FEBS Letter 189, 102-108; 1985). All above factors elute from a heparin-Sepharose column at pH 7.0 with about 1.6 M NaCl, have apparent pIs of about 8-10 and share high homology in the amino acid sequence.

Although the use of heparin has facilitated the initial purification of such factors, the identification of the genes and cDNAs coding for these mitogens (Abraham J.A. et at.; EMBO J. 5, 2523-2528; 1986. Kurokawa T., et at., FEBS Letter 213, 189-194, 1987) and the development of E. coli expression systems (Kurokawa T. et at.; FEBS Letter 213, 189-194, 1987), have allowed to better understand of the relationship within these closely related factors.

The genetic studies suggest that there is only one basic mitogen that is bFGF; differences among the molecules isolated from different tissues reflect variable N-terminal truncation due to post-translational processing (Gospodarowicz D. et at.; Endocr.Rev. 8, 95-114, 1987. Esch F. et at.; PNAS USA 82, 6507-6511, 1985).

Where studied, these differences in length have been found to have no significant effect on either mitogenic activity or heparin affinity (Gospodarowicz D., et at.; Endocr. Rev. 8, 95-114, 1987).

It has been postulated that the presence of N-terminally extended forms may be important in tissue-specific regulation. Basic FGF has been shown to have a wide spectrum of activity. It increases DNA synthesis and cell division in cells of mesodermal and neuroectodermal origin; regulates proteins synthesis and secretion (e.g., plasminogen activator); affects cell motility and migration (e.g., glia); modifies differentiated function, especially of neural and endocrine cells (e.g., pituitary); may influence cell survival (e.g., damaged neurons) and senescence (e.g., adrenal cortical cells).

The mitogenic, chemotactic and differentiating properties of the molecule suggest that it is involved in embryonic development and it is probably a major trophic factor operating at all stages of embryogenesis (Slack J., et al.; Nature 326, 197-200, 1987).

Control of angiogenesis by bFGF is expecially important in reproductive tissues, where it may also control growth and differentiation of granulosa cells in the corpus luteum in preparation for either luteinisation or artresia (Gospodarowicz D., Sem. Repro. Endocr., 7, 21-39; 1989).

In addition, bFGF may help to regulate other highly vascular endocrine organs such as pituitary testis, thyroid, and adrenal cortex since the protein is localised immunocytochemically in these tissues and can modulate hormone synthesis (Baird A. and Bohlen P. Fibroblast growth factors. In: Sporn MB, Roberts AB eds. Handbook of experimental pharmacology. Vol. 95/1. Peptide growth factors and their receptors I. Berlin: Springer, 1990: 369-418).

The variety of bFGF functions, as well as its wide tissue distribution, suggests that this mitogen is of broad physiological significance and of potential clinical value. Among the many possible clinical uses, bFGF can find an application in accelerating wound healing (especially in deep dermal lesions) in the revascularization of ischaemic tissues, in the regeneration of damaged neural tissues, in enhancing the success of tissue transplantation and of bone graft healing.

We have now developed derivatives of human bFGF and its functionally equivalent analogues, characterized in that they show a considerably increased biological activity and stability when compared to the native molecules. This finding is of major importance in the therapeutical applications of bFGF depicted above.

Detailed description of the invention

The present invention relates to derivatives of human basic fibroblast growth factor (bFGF) and its functionally equivalent analogues, as well as to a process for their production.

Human bFGF is a single chain, non-glycosylated protein having a molecular weight of approximately 16,500 daltons which was originally reported to be present in, and was extracted from, human brain (Gimenez-Gallego, G. et al; Biochem.Biophys. Res.Comm. 135, 541-548; 1986).

In EMBO Journal vol. 5, no. 10, pp. 2523-2528, 1986 and PCT International Publication No. WO/01728, it is shown that the human bFGF constituent aminoacids were deductively specified on the basis of the clone which was produced by cloning cDNA of human bFGF using bovine bFCF as a probe.

In addition, FEBS Letter vol. 213, p. 189, 1987 describes the production of human bFGF by cultivation of a transformant obtained by cloning cDNA of human bFGF.

The nucleotide sequence coding for human bFGF predicts a 155 amino acid translation product, but several shorter and longer forms, differing in length at the N-terminus, have been isolated from different tissues. Even the recombinantly produced molecule often appears as a mixture of different forms due to partial proteolytic degradation at the N-terminus.

Despite these differences in length all these forms appear to retain the biological activity of bFGF.

The common 146 amino acid sequence of human bFGF is represented by the following formula:

```
  1                                          10
Pro Ala Leu Pro Glu Asp Gly Gly Ser Gly Ala Phe Pro Pro Gly His
              20                                      30
Phe Lys Asp Pro Lys Arg Leu Tyr Cys Lys Asn Gly Gly Phe Phe Leu
                              40
Arg Ile His Pro Asp Gly Arg Val Asp Gly Val Arg Glu Lys Ser Asp
      50                                      60
Pro His Ile Lys Leu Gln Leu Gln Ala Glu Glu Arg Gly Val Val Ser
                      70                                      80
Ile Lys Gly Val Cys Ala Asn Arg Tyr Leu Ala Met Lys Glu Asp Gly
                                      90
Arg Leu Leu Ala Ser Lys Cys Val Thr Asp Glu Cys Phe Phe Phe Glu
              100                                     110
Arg Leu Glu Ser Asn Asn Tyr Asn Thr Tyr Arg Ser Arg Lys Tyr Thr


Ser Trp Tyr Val Ala Leu Lys Arg Thr Gly Gln Tyr Lys Leu Gly Ser
      130                                     140
Lys Thr Gly Pro Gly Gln Lys Ala Ile Leu Phe Leu Pro Met Ser Ala
      146
Lys Ser
```

This sequence may have present an N-terminal extension including the whole or a part of the following 11 aminoacid sequence.

    i) Gly-Thr-Met-Ala-Ala-Gly-Ser-Ile-Thr-Thr-Leu

    for example, in particular, the following 9 aminoacids:

    ii) Met-Ala-Ala-Gly-Ser-Ile-Thr-Thr-Leu

    or the following 8 aminoacids

    iii) Ala-Ala-Gly-Ser-Ile-Thr-Thr-Leu

or the following 7 aminoacids

iv) Ala-Gly-Ser-Ile-Thr-Thr-Leu.

Even shorter forms of the 146 aminoacid long molecule were isolated; these are N-terminal deleted bFGFs lacking of one or more of the aminoacid residues.

Human bFGF analogues with comparable biological activities are also included in the definition of human bFGF according to the invention.

An analogue may be represented by a mutein derived from the above indicated forms characterized in that its aminoacid sequence differs from the natural sequence by way of amino acid substitutions, deletions, inversions and/or additions. Examples of basic FGF analogues may be found in the published European Patent Application No. 363675.

All the different forms of human bFGF and its analogues described above may also be amidated at their C-terminal end. Also mixtures of two or more different forms of human bFGF are to be considered as functional equivalents of the natural molecule.

In particular, for example, a mixture of two different forms having a N-terminal extension of 7 and 8 aminoacid residues respectively, with respect to the natural 146 aminoacid long molecule, is considered in this specification.

In this specification this mixture is indicated with the internal code FCE 26184 which represents a human bFGF of 153-154 amino acids, precisely an approximately 50:50 mixture of a) a 153 aminoacid molecule having the sequence of 146 aminoacids shown previously for human bFGF and a N-terminal extension of 7 aminoacids as indicated in point (iv) on page 5, and b) a 154 aminoacid molecule having the sequence of 146 aminoacids shown previously for human bFGF and a N-terminal extension of 8 aminoacids as indicated in point (iii) on page 5.

Thus, the term "human basic fibroblast growth factor" as used in the present patent application includes the natural proteins as well as the above indicated analogues and mixtures.

Natural human basic FGF contains four cysteine residues, but the number of disulfide bonds if any, is unknown.

In the course of the production of bFGF by recombinant DNA technique, heterogeneous conformations were found in Escherichia coli extract containing a high concentration of bFGF.

It is known that cysteine containing proteins produced in bacteria often form incorrect intramolecular disulfide bonds which can inhibit biological function (see human interleukin-2; Wang et al. Science (1984) 224, 1431-1433 and human fibroblast interferon; Mark et al. Proc. Natl. Acad. Sci. (USA) (1984) 81; 5662-5666).

Modifications of one or more of the cysteine residues present in the native bFGF proteins may minimize incorrect disulfide bridge formation, eliminate the need for use of reducing agents to stabilize the bFGF protein, and hence reduce multimerization or incorrect disulfide bonds thereby increasing the recoverable yield of the recombinantly produced analog, increasing the uniformity of the bFGF preparation by maintaining it over time in a monomeric form, improving its shelf stability and prolonging its half life when applied to wounds.

In a more recent work Seno et al. [Biochem. Biophys. Res. Comm. 151(2), 701-708, 1988] individually changed to serine each of the four cysteines present in human bFGF thus finding that the substitution of the cysteine residues in position 69 and 87 with serine, reduced the heterogeneity recognized as several peaks of bFGF eluted from a heparin affinity column or from reversed phase HPLC. These data suggest that the cysteines at these positions are exposed to the surface of the molecule to form disulfide inter- or intra-molecular bonds that induce heterologous conformations.

Seno et al. also reports that the serine 69, 87 analog does not show any increased biological activity with respect to that of natural sequence bFGF.

Furthermore Fox, G.M. et al. [J.Biol.Chem. 263, 18452-18458, 1988] suggest that the cysteines at positions 25 and 92 of the recombinantly produced human bFGF are probably joined by an intra-molecular disulfide bond.

Very recently the International Patent Application Publication no. PCT WO90/13310 presented data concerning the stabilization of bFGF by thiolation of its cysteine residues with an agent capable of forming an S-S bond with at least one cysteine contained in the bFGF molecule.

According to the results obtained using glutathione disulfide, as protective agent the derivatized bFGF has shown an enhanced stability and an increased resistance to multimerization in a short term (5 days) incubation assay.

We have now synthetized and isolated the same derivatized product specifically described in PCT WO90/13310 and we have tested their stability in a long term (25 days) incubation assay.

Following storage at room temperature or incubation at alkaline pH, the S-thiolated bFGF of PCT WO90/13310 was found susceptible to further modifications of the molecule, probably due to reshuffling of the disulfide bridges forming a mixture of molecular species where at least an intramolecular S-S bond between Cys 96 and Cys 101 occurred.

Similar results were obtained also for the cysteine derivatized compound disclosed in PCT WO90/13310.

We have now succeeded in irreversibly and chemically derivatizing the recombinantly produced human bFGF in such a way to obtain a derivatized molecule free of aggregation due to dimer formation, which has been found not only to be more easily recovered and more stable also in a long period incubation assay. but also, and above all, unexpectedly much more active than the corresponding unmodified protein.

One object of the invention is to provide a recombinant human basic fibroblast growth factor or a functionally equivalent analog thereof in which at least one of the four cysteine amino acid residues is derivatized with a derivatizing agent capable of forming a direct S-C bond with the cysteine residue.

In particular, an object of the present invention is to provide a recombinant human basic fibroblast growth factor or a functionally equivalent analog thereof in which the two cysteines corresponding to those present in positions 69 and 87 of the 146 aminoacid form of the natural human basic fibroblast growth factor, are derivatized with a derivatizing agent capable of forming a direct S-C bond with the cysteine residues.

More particularly, an object of the present invention is to provide a derivatized recombinant human bFGF or a functionally equivalent analog thereof in which the two cysteines corresponding to those present in positions 69 and 87 of human bFGF are derivatized, e.g., with a substituent selected from the group consisting of $(-CH_2COOH)$, $[-CH(COOH)(CH_2)_xCOOH]$, $(-CH_2CONR_3R_4)$, $(R_5)$, $[-(CH_2)_nSO^-_3]$, $(-CHCH_2CONR_3CO)$, $[-(CH_2)_mNR_3R_4]$ and $(-CH_2OCOCH_2R_5)$ wherein $R_3$ and $R_4$ are each H, $[-(CH_2)_xCOOH]$, $[CH(COOH)(CH_2)_xCOOH]$ or $C_1$-$C_6$ alkyl; $R_5$ is $C_1$-$C_6$ alkyl or $C_1$-$C_4$ alkoxymethyl, n is zero or an integer of from 1 to 4, m is an integer of from 2 to 4 and x is an integer of from 1 to 3.

The derivatized bFGFs provided by the invention may be prepared, by known methods according to procedures commonly used in the organic chemistry, by reacting the underivatized bFGF molecule with a suitable reagent carrying the desired derivatizing moiety, e.g. among those listed hereabove.

Thus, for example a derivatized recombinant human bFGF, according to the invention may be obtained by a method comprising dissolving a recombinant human bFGF in water or in a buffered aqueous solution containing EDTA; adjusting the pH of said solution to a value of, e.g., about 8.0; treating said pH adjusted solution with the desired derivatizing agent; eliminating from the resulting mixture the derivatizing agent in excess by loading the said mixture on a Mono S column and extensively washing the column with a buffered solution; eluting the derivatized human bFGF with a linear gradient of sodium chloride and desalting the derivatized human bFGF by passing it through a Sephadex G-25 column.

The employed derivatizing agent is chosen according to the kind of derivatization which it is desired to obtain.

Thus, for obtaining a derivatized bFGF wherein the -SH group of the cysteine residues is derivatized to -S-CH$_2$-COOH group the derivatizing agent is chosen, e.g., from a haloacetic acid, in particular iodoacetic acid, or a salt thereof, e.g., an alkali metal salt, especially the sodium salt thereof.

A corresponding haloacetamide may be used as the derivatizing agent when it is desired to obtain a derivatized bFGF wherein the -SH group of the cysteine residues is changed to a -S-CH$_2$-CONH$_2$ group.

As it will be evident to the skilled in the art, an alkalimetaltetrathionate, a $C_1$-$C_4$ alkyl methanethiosulphonate or a $C_1$-$C_6$ alkylsulfone may be derivatizing agents useful for obtaining other derivatized bFGFs among those specifically mentioned herein before.

A particularly preferred derivatized bFGF according to the invention is human bFGF wherein the -SH groups of the cysteines 69 and 87 are derivatized to -S-CH$_2$-COOH groups.

When the human bFGF is the 146 amino acid long form of the molecule, the derivatized compound will be referred to herebelow as CM-bFGF which means carboxymethylated bFGF.

Another preferred derivatized bFGF according to the invention is human bFGF wherein the -SH groups of the cysteines 69 and 87 are derivatized to -S-CH$_2$-CONH$_2$. Again when the human bFGF is the 146 amino acid long form of the molecule, the derivatized compound will be referred to herebelow as CAM-bFGF which means carboxyamidomethylated bFGF.

The FGFs starting compounds for the derivatives of the invention are generally obtained by recombinant DNA techniques with well known procedures such as those described in the previously mentioned International Patent Application No. PCT WO87/01728 or in the European Patent Application No. 363675.

Advantageously, the derivatized human bFGFs of the present invention are useful in the same fields of application of the natural bFGF such as in the revascularization of ischaemic tissues, in the regeneration of damaged neural tissues, in the healing of wounds, ulcers and burns or as adjunct to other therapeutic regimens, for example during transplantation.

The derivatized human bFGFs of the invention can be administered in the form of pharmaceutical compositions comprising one or more different forms of derivatized bFGF and one or more pharmaceutically acceptable excipients.

In practice these compositions may be administered by parenteral injection of phosphate buffered solution or, alternatively, as sustained release compositions of gels, pastes, microspheres and the like.

When administered as a phosphate buffered solution, the pharmaceutical compositions of derivatized human bFGF may comprise pharmaceutically acceptable excipients, e.g., carriers and/or diluents.

The derivatized human bFGF may be administered as such or as pharmaceutically acceptable salt of pharmaceutically acceptable inorganic acids, e.g., hydrochloric, hydrobromic, sulphuric and phosphoric acid, or pharmaceutical acceptable organic acids, e.g., acetic, maleic, malic, citric, succinic and tartaric acid. The pharmaceutical compositions of the invention can be prepared with known techniques according to conventional procedures.

The present invention is further illustrated by the examples set forth hereinbelow.

## Preparation example 1

Preparation of FCE 26184.

The construction of the synthetic DNA sequence for bFGF and of the expression plasmid carrying such a sequence was performed according to the procedure described in EP 363675. The fermentation and purification process was carried out as follows:

### (a) Fermentation process

A bacteria strain, E. coli type B, from the Institute Pasteur collection, was transformed with a plasmid carrying both the human gene coding for bFGF and the gene for tetracycline resistance. This transformed strain was used for the production of recombinant non-glycosylated h-bFGF (human bFGF). A Master Cell Bank (W.C.B.) (79 vials stored in liquid nitrogen at -190°C) of this strain were prepared. The content of one vial of W.C.B. was used as the inoculum for the fermentation phase.

The fermentation process was carried out in 10 l fermentors filled with 4 l of culture medium.

Tetracycline hydrochloride was added to the medium in order to maintain the conditions of strain selection.

After 20 hours of growth at 37°C the final biomass was 42 ± 2 g/l dry weight, and the production of bFGF was 2500 ± 500 mg/l as measured by comparative gel electrophoresis.

Enrichment in pure oxygen was required during the fermentation phase in order to allow a large bacterial growth.

### (b) Initial purification

The cells (microorganisms) were separated from the total fermentation broth by centrifugation. The resulting pellet was resuspended in a sodium phosphate buffer containing sodium chloride. A minimum of 3 passages through a high pressure homogenizer were necessary for efficient cell breakage. The resulting cell lysate was clarified by centrifugation and the supernatant was collected for further processing.

### (c) Purification

The clarified supernatant was loaded on a column of Sepharose (Trade Mark) S Fast Flow (cation exchanger) and the product was eluted from this column using a gradient of increasing sodium chloride concentrations in a phosphate buffer (Trade Mark). The product was further purified on a column of Heparin Sepharose 6 B by eluting with a gradient of increasing sodium chloride concentration in a phosphate buffer. Finally a buffer exchange was made on a Sephadex (Trade Mark) G25 resin to obtain the product in the bulk product buffer (Sodium phosphate -EDTA).

### (d) Column sanitization

Sepharose S Fast Flow and Sephadex G25 columns were sanitized by washing with sodium hydroxide solutions.

Heparin Sepharose was washed alternatively with solutions at pH = 8.5 and pH = 5.5 containing 3M sodium chloride.

Preparation example 2

Preparation of human bFGF, 146 amino acid form.

A recombinantly produced protein corresponding to the natural human bFGF (146 amino acids) can be obtained, for example, following the method described in the published International
Patent Application No. WO87/01728.

Example 1

Preparation of human carboxymethylated bFGF (CM-FGF)

To a solution of 100 mg of recombinant human basic Fibroblast Growth Factor 146 amino acid long form (rhbFGF) in 110 ml of 25 mM phosphate buffer pH 8.0/5 mM EDTA, was added 400 mg of iodoacetic acid in 110 ml of the same buffer. The reaction was allowed to stand at room temperature for two hours in the dark. The reaction mixture was then directly loaded on a MonoS column (HR 10/10, Pharmacia) equilibrated in 25 mM phosphate buffer pH 7.5. In order to eliminate the reagent excess, the column was extensively washed with the equilibration buffer and the carboxymethylated rhbFGF (CM-FGF) was eluted with a linear gradient from 0 to 1 M NaCl in 25 mM phosphate buffer pH 7.5. The CM-FGF containing fractions were desalted on a Sephadex G-25 column (Pharmacia) equilibrated in 10 mM phosphate buffer pH 6.0/0.1 mM EDTA.

Example 2

Preparation of human carboxamidomethylated bFGF (CAM-FGF)

To a solution of 100 mg of recombinant human basic Fibroblast Growth Factor 146 amino acid long form (rhbFGF) in 110 ml of 25 mM phosphate buffer pH 8.5/5 mM EDTA, was added 400 mg of iodoacetamide in 110 ml of the same buffer. The reaction was allowed to stand at room temperature for three hours in the dark. In order to remove the fine precipitate formed during incubation the reaction mixture was centrifuged and the clean supernatant was directly loaded on a MonoS column (HR 10/10, Pharmacia) equilibrated in 25 mM phosphate buffer pH 7.5.

In order to eliminate the reagent excess, the column was extensively washed with the equilibration buffer and the carboxyamidomethylated rhbFGF (CAM-FGF) was eluted with a linear gradient from 0 to 1 M NaCl in 25 mM phosphate buffer pH 7.5. The CAM-FGF containing fractions were desalted on a Sephadex G-25 column (Pharmacia) equilibrated in 10 mM phosphate buffer pH 6.0/0.1 mM EDTA.

Example 3

SH titration of rhbFGF derivatives

CM-FGF, CAM-FGF and rhbFGF were subjected to SH titration carried out in 6M guanidine hydrochloride, 0.1 M Tris-HCl, 1.5 mM EDTA, pH 8.0 using 5,5'-dithiobis-(2-nitrobenzoic acid) (DTNB) essentially according to Habeeb, A.F.S.A. 25 Methods Enzymol. 457-465 (1972).

The protein concentration of the different solutions of rhbFGF and its derivatives was estimated by amino acid analysis. The SH titration resulted in detection of exactly 2 moles of free sulfhydryl per molecule in the case of all the derivatized molecules and 4 moles of free sulfhydryl per molecule in the case of rhbFGF. This suggested that whereas purified rhbFGF had all the four sulfhydryl groups free, CM-FGF and CAM-FGF had two free sulfhydryl groups and two derivatized cysteines.

Example 4

Protease treatment and peptide mapping of CM-FGF

In order to know which cysteines are alkylated in the CM-FGF, peptide mapping was attempted with two different proteolytic treatment, i.e. with trypsin and V8 Staphilococcus aureus protease.

For comparison, peptide mapping was also performed under the same conditions on the native recombinant bFGF and on a derivative with all cysteine alkylated, pyridylethylated-CM-FGF (PE-CM-FGF).

Methods

For each digestion, 20-50 µg of different bFGF samples (native or modified) were used in a final volume of 100 µl of buffer.

The resulting peptides were separated by RP-HPLC and the cysteine containing peptides were identified by means of N-terminal sequence analysis.

**Trypsin digestion** - bFGF samples were digested with TPCK treated trypsin at 37°C for 24 hours in 0.1 M ammonium bicarbonate at pH 8 with an enzyme-to-substrate ratio of 1:50 (w/w).

**V8 protease digestion** - Digestion with V8 protease was carried out at 37°C in 50 mM ammonium acetate pH 4 for 16-24 hours with E:S = 1:25 (w/w).

**RP-HPLC** - Digests were subjected to reversed-phase HPLC on a µBondapak C18 column (Waters; 3.9 x 300 mm, 10 µ) eluted with a linear gradient from 5% to 65% of acetonitrile in 0.1% TFA in 60 or 90 min., at a flow rate of 1 ml/min. Samples were injected by means of a Water Wisp autosampler; for further analysis fractions were manually collected, monitoring at 220 nm.

**N-terminal sequence analysis** - Automated N-terminal sequence analysis was performed on a gas-phase sequencer mod. 470A or on a pulsed liquid phase sequencer mod 477A (Applied Biosystems) equipped with an on-line HPLC PTH-Analyzer mod. 120A (Applied Biosystems).

For analysis, standard programs furnished by manufacturers were used with little modifications.

Results

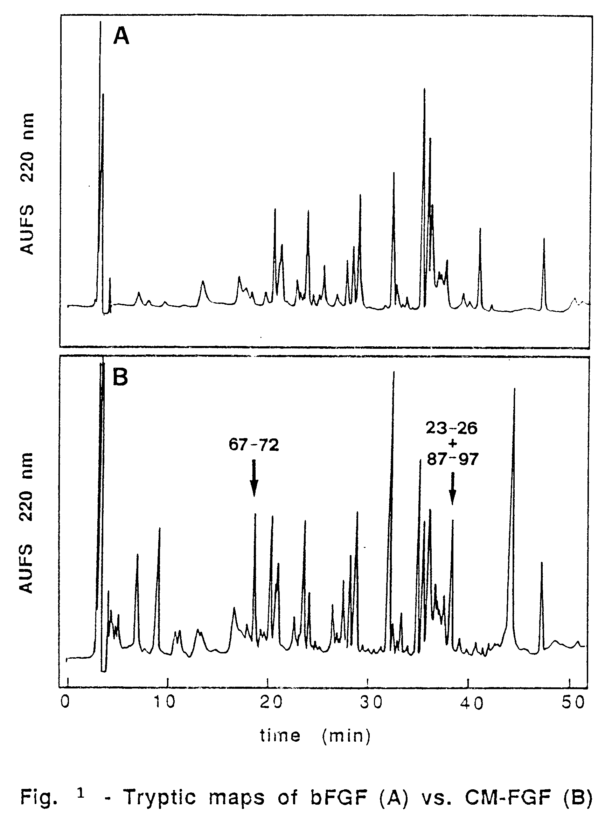

Trypsin mapping was tried as a first attempt to identify which cysteine residues are alkylated in the CM-FGF; on the basis of the primary structure of bFGF and trypsin specificity, three cysteine containing peptides are expected, namely 23-26 (Cys 25), 67-72 (Cys 69), 87-97 (Cys 87 and 92). Comparison of tryptic maps of CM-FGF vs. bFGF (Fig. 1) shows maily two different peaks, which, subjected to aminoacid sequence analysis, resulted in peptide 67-72 with the cysteine 69 carboxymethylated (CM-Cys) and peptides 23-26 and 87-97 linked by a disulphide bridge, where only Cys 87 was identified as PTH-CM-Cys. These findings suggest that the two free SH initially present in the CM-FGF (see example 5) tend to form an intra-chain disulfide bond when incubated for prolonged time at 37°C under alkaline conditions usually employed for trypsin digestion. Actually, after incubation of CM-FGF at basic pH for several hours, essentially no free -SH can be titrated with DTNB.

As a proof that only Cys 69 and 87 are carboxymethylated in the CM-FGF, V8 protease digestion was performed at acidic pH on native recombinant bFGF, CM-FGF and PE-CM-FGF. The latter was obtained after a further step of alkylation with 4-vinyl-pyridine under non reducing conditions with 6M Guanidine hydrochloride Gnd-HCl / 0.25 M Tris-HCl essentially according to Dupont, D. et al. (Derivatizer-Analyzer User Bulletin No. 1. Applied Biosystems, Inc., 1987), and subsequent C4-HPLC purification following the conditions described for stability experiments (see example 7).

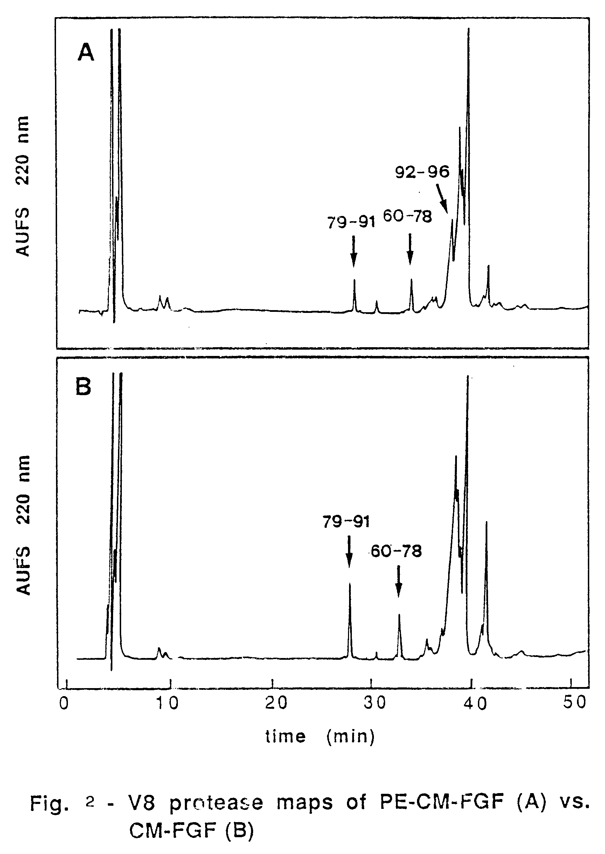

V8 protease digestion of CM-FGF vs. PE-CM-FGF (Fig. 2) allows straight identification of CM-Cys 69 in peptide 60-78, CM-Cys 87 in peptide 79-91 and PE-Cys 92 in peptide 92-96, while Cys 25 was proved to be susceptible to pyridylethylation only by direct N-terminal sequence of PE-CM derivative of bFGF, likely because the expected peptide 5-45 is obtained in low yield in V8 peptide mapping.

These results indicate that Cys 69 and 87 are quantitatively carboxymethylated in the CM-FGF while Cys 25 and 92 remain in the reduced form.

Example 5

Stability of CM-FGF at 25°C and 4°C

A solution of CM-FGF and rhbFGF at a concentration of 300 µg/ml in 25 mM phosphate buffer pH 7.5/0.125 M NaCl/0.1 mM EDTA was filtered through a Millipore sterile Millex-0.22 µ filter unit and incubated at 25°C and 4°C.

Some aliquots of each solution were taken after one, two and three weeks, subjected to reverse phase HPLC using a Vydac C4 column (CTR 214 C4; 0.46 x 25 cm) and eluted with a linear gradient of 10%-90% Acetonitrile in 0.1% TFA at a flow rate of 1ml/min.

Resulting amount of proteins in solution from HPLC analysis was compared with the initial amount and a stability profile reported.

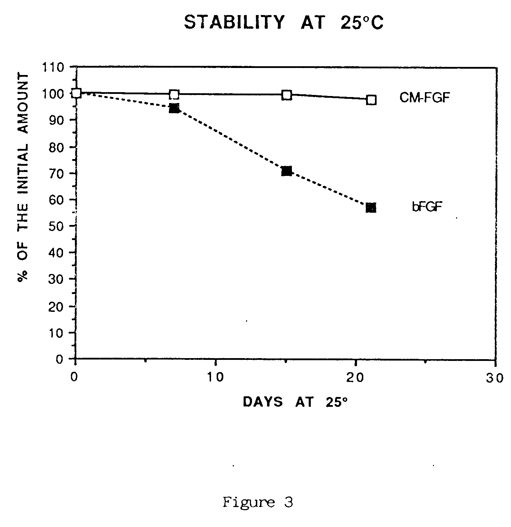

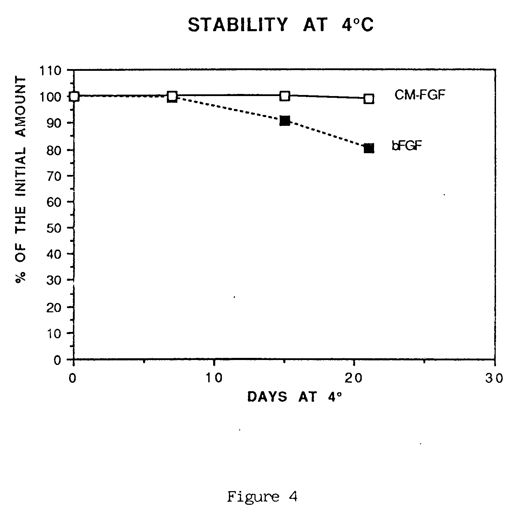

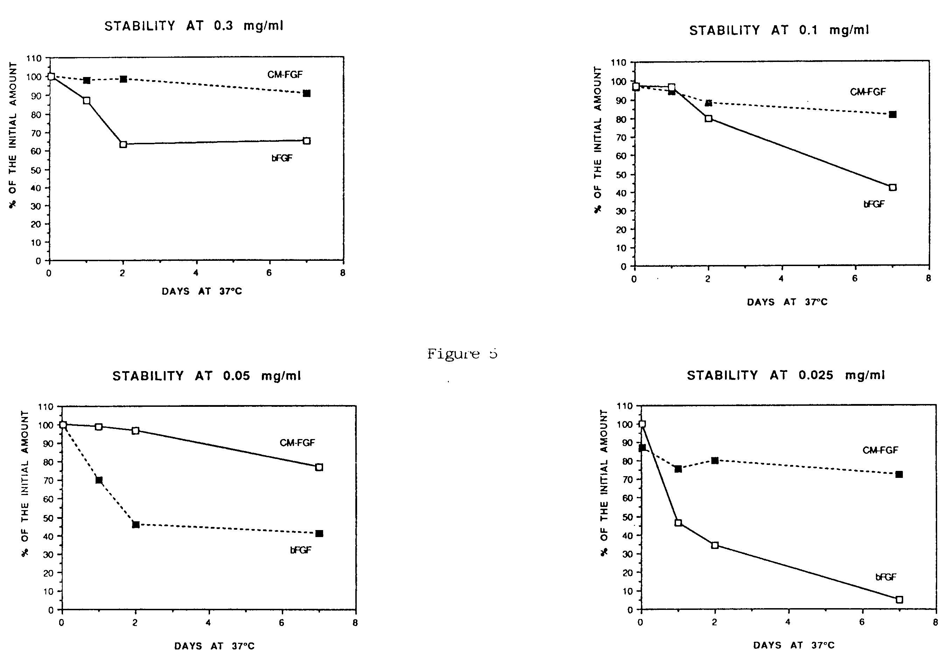

In Figure 3, percent of the initial amount of CM-FGF and bFGF was plotted versus incubation time at 25°C.

Figure 4 shows the same after three weeks incubation time at 4°C.

The results of the incubation at 25°C demonstrated that whereas only 57% of the initial amount of rhbFGF was available in solution after 21 days, 98% of CM-FGF was avaliable after the same interval time. At 4°C incubation temperature after three weeks the recovery of CM-FGF and bFGF was 99% and 80% respectively.

Thus, in the case of bFGF the carboxymethylation of the two cysteines led to a derivative with an increased stability in vitro against degradations that mainly involved a modification of the two most reactive sulfhydryl in the molecule.

Example 6

Stability of CM-FGF at different concentrations at 37°C

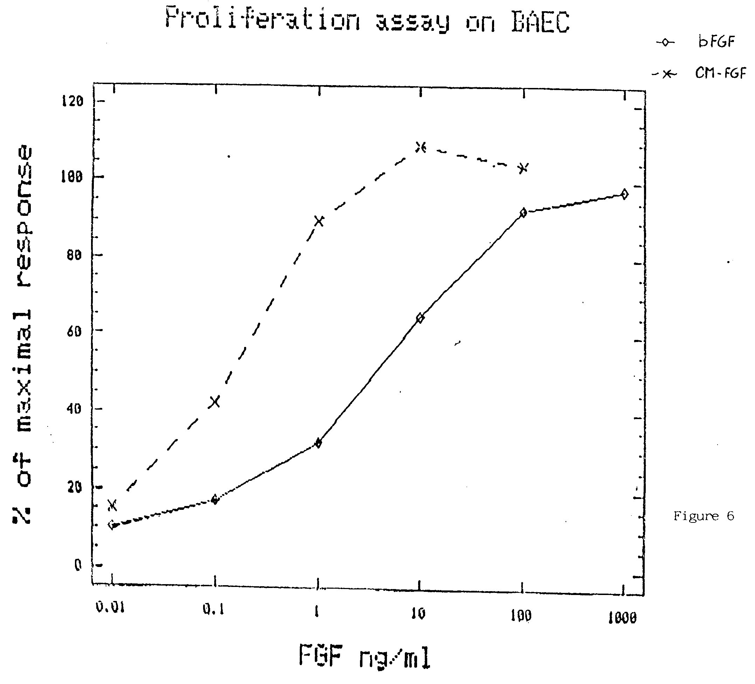

In order to mimic the behaviour in physiological conditions CM-FGF and rhbFGF were both incubated at 37°C at a protein concentration of 300, 100, 50 and 25 $\mu$g/ml in 25 mM phosphate buffer pH 7.5 / 0.125 M NaCl / 0.1 mM EDTA in sterile conditions. After 1,2 and 7 days an aliquot of each solution was taken and subjected to an HPLC analysis using the same conditions described above. Figure 5 shows the percent of the initial amount of CM-FGF vs. rhbFGF at different times for each protein concentration in the incubation buffer. The actual amount of rhbFGF and CM-FGF after 7 days at the different concentrations compared with the initial quantity of protein was:

|  | 0.3 mg/ml | 0.1 mg/ml | 0.05 mg/ml | 0.025 mg/ml |
|---|---|---|---|---|
| rhbFGF amount | 65.3% | 42.0% | 40.1% | 5.3% |
| CM-FGF amount | 90.8% | 82.0% | 76.3% | 72.3% |

At an initial concentration of 0.025 mg/ml the rhbFGF measured in solution after 7 days incubation time at 37°C was only 5% of the total initial amount whereas 72.3% of CM-FGF was recovered at that time. The sample of rhbFGF and CM-FGF used to evaluate the stability by HPLC were also assayed for their biological activity according to the in vitro mitogenesis assay in 3T3 cells reported below. The biological activity data paralleled the analytical data reported in the above table, confirming a considerable loss of activity for rhbFGF and much less so for CM-FGF. Due to the increased stability of carboxymethylated bFGF even at very low concentrations new formulative and therapeutic approaches could be seen. The likely higher bioavaliability of the product in vivo could lead to a lower therapeutic dose associated to decreased side effects.

Example 7

Biological activity of CM-FGF

FGF was originally described as a mitogen in 3T3 fibroblasts and subsequently was found to be a potent mitogen for other cells, including endothelial cells. For this reasons, in order to compare the mitogenic activity of CM-FGF vs. rhbFGF, they were assayed in Balb/c 3T3 fibroblasts, and in bovine aortic endothelial cells. Further, an in vitro competitive receptor binding assay on isolated cells was used to evaluate the affinity of the modified bFGF molecule i.e. CM-FGF as compared to the parent intact recombinant bFGF.

Widely reported in literature is also the ability of bFGF to strongly bind heparin. We have therefore compared CM-bFGF and rhbFGF for this capacity. The binding to heparin was determined using heparin affinity HPLC analysis.

Methods

Mitogenesis assay on Balb/c 3T3 cells for stability evaluation.

A31-1-1 cells, a cell line established from Balb/c mouse embryos, were grown in Eagle's minimal essential medium (MEM) supplemented with 10% fetal bovine serum (FBS), 100 units/ml of penicillin and 100 $\mu$g/ml of streptomycin.

The mitogenic activity was determined by measuring the incorporation of $^3$H-thymidine into cellular DNA.

On day 1 of the assay, subconfluent cultures were detached by trypsinization and plated ($1\times10^4$ cells in 200 $\mu$l/well) in 96 well micro-titre plates. The "seeding medium" contained a reduced amount (3% v/v) of fetal bovine serum (FBS). The cultures were incubated at 37°C, 5% $CO_2$ and 95% humidified atmosphere; 22 h later, the "seeding medium" was replaced with a "starvation medium" consisting of Eagle's MEM with 0.1% (v/v) FBS and incubation was continued for a second period of 22 hrs. Thereafter, the cells were washed once with phosphate buffered saline (PBS) and subsequently refed with fresh serum-free "exposure medium" consisting of MEM supplemented with 0.1% bovine serum albumin (BSA) in which samples were diluted at the concentrations to be tested.

8 hrs after the beginning of the exposure, Balb/3T3 cells were labelled by addition of $^3$H-thymidine (sp.ac. 20 Ci/mmol; 0.25 $\mu$Ci/well) and the exposure was continued for an additional 16 hrs. Thereafter the medium was removed, and cells in each well were washed 2 times with $Ca^{++}/Mg^{++}$ - free PBS. Then PBS containing 0.02% EDTA and 0.25% trypsin (100 $\mu$l/well) were added and the plates were incubated for 10' to achieve complete detachment of the cultures. Cells in each well wore collected on glass-fiber filters using the cell-harvester and wells were washed 15 times with distilled water to assure both adequate cell-lysis and washing to remove the unbound $^3$H-thymidine. Airdried filters were placed into vials containing 4 ml of liquid scintillation cocktail.

The radioactivity retained on the filters was determined in a scintillation counter.

Six replicates were made for each experimental condition.

Proliferation assay on BAE cells for potency estimation.

Cell were plated at 2500 cells/well in 96 microtiter plates in Dulbecco's modified Eagle's medium (DMEM) supplemented with antibiotics (penicillin 100 U/ml, streptomycin 100 $\mu$g/ml) and with FBS (13% v/v). The cells were allowed to attach for a period of 6 hours. Thereafter, the medium was removed and the samples diluted at desired concentration into experimental media, (DMEM supplemented with 0.5% FBS, 0.1% BSA) were added to cells.

The cultures were incubated for 3 days at which time they were fixed with 10% phosphate buffered formalin and stained with 0.5% crystal violet for 10'. After staining, wells were thoroughly washed to remove unincorporated stain. Methanol (95% v/v; 100 $\mu$l/well) was added to each well and incubated for 20' to extract the dye retained by the cultures to an extent proportional to the number of cells grown/well.

Plates were than transferred for automatic reading to a spectrophotometric microplate reader equipped with a 540 nm filter. Four replicates were made for each experimental condition.

High affinity receptor binding assay for affinity estimation. Confluent BHK cells were seeded in 24 well plates in DMEM supplemented with 10% FBS supplemented with antibiotics (penicillin 100 U/ml, streptomycin 100 $\mu$g/ml).

Cells were incubated for 2 hours at 37°C in serum-free DMEM with 0.1% gelatin and 20 mM Hepes (binding buffer) prior to the binding assay.

125 Iodine-labelled bFGF (sp. ac. 1000 Ci/mmol; 20 nCi/well) and the test compounds diluted in binding buffer, were simultaneously added to the cells.

Binding was carried out at 4°C for 3 hours, then the cells were washed twice with cold $Ca^{++}/Mg^{++}$ free PBS, and twice with 2M NaCl pH 7.5. The cell-bound radioactivity was collected with 0.5 N NaOH, transferred into vials and measured by gamma-counting.

Heparin affinity assay.

Protein samples of CM-bFGF and rhbFGF were injected into a Shodex AF pak HR-894 column (0.8 x 5 cm, Showa Denko, Tokyo, Japan) equilibrated in 25 mM phosphate buffer pH 7.5 and eluted with a linear gradient (0-2 M) of NaCl in the same buffer at a flow rate of 1 ml/min.

Affinity of each sample to heparin was referred to the molarity of NaCl required to elute the particular protein from the column, as estimated by conductivity.

Results.

The in vitro biological activity of CM-FGF as compared to rhb-FGF was studied in a proliferation assay with BAE cells.

Data reported in figure 6 show that in this in vitro system CM-FGF is approximately 25 fold more potent as the parent rhb-FGF.

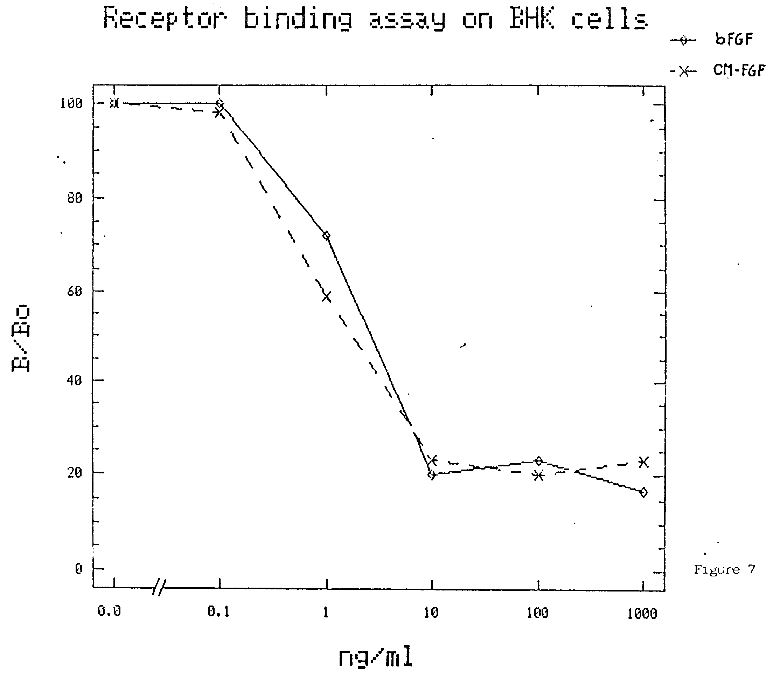

On the contrary the competitive receptor binding assay performed on BHK cells did not show any statistically significant difference for the binding of the two molecules to these cells (Fig. 7).

From the heparin affinity assay CM-bFGF and rhbFGF were found to have similar affinities for immobilized heparin, both eluting at 1.1 M NaCl as single peaks.

## Claims

1. A recombinant human basic fibroblast growth factor or a functionally equivalent analog thereof, in which at least one of the four cysteine amino acid residues is derivatized with a derivatizing agent capable of forming a direct covalent S-C bond with the cysteine residue.

2. A recombinant human basic fibroblast growth factor or a functionally equivalent analog thereof according to claim 1, in which the two cysteines corresponding to those present in positions 69 and 87 of the 146 amino acid form of the natural human basic fibroblast growth factor are derivatized with a derivatizing agent capable of forming a direct covalent S-C bond with the cysteine residues.

3. A recombinant human basic fibroblast growth factor or a functionally equivalent analog thereof according to claim 1 or 2 wherein the cysteine is derivatized with a substituent selected from the group consisting of ($-CH_2COOH$); [$-CH(COOH)(CH_2)_xCOOH$]; ($-CH_2CONR_3R_4$); ($R_5$); [$-(CH_2)_nSO^-_3$]; ($-CHCH_2CONR_3CO$); [$-(CH_2)_mNR_3R_4$] or ($-CH_2OCOCH_2R_5$); wherein $R_3$ and $R_4$ are each H, [$-(CH_2)_xCOOH$]; [$-CH(COOH)(CH_2)_xCOOH$]; $C_1$-$C_6$ alkyl; $R_5$ is $C_1$-$C_6$ alkyl or $C_1$-$C_4$ alkoxymethyl; n is zero or an integer of from 1 to 4; m is an integer of from 2 to 4; and x is an integer of from 1 to 3.

4. A recombinant human basic fibroblast growth factor or a functionally equivalent analog thereof according to claim 2 wherein the two cysteines are derivatized with ($-CH_2COOH$).

5. A recombinant human basic fibroblast growth factor or a functionally equivalent analog thereof according to claim 2 wherein the two cysteines are derivatized with ($-CH_2CONH_2$).

6. A method for obtaining a derivatized human basic fibroblast growth factor or functionally equivalent analog thereof, said method comprising: dissolving said recombinant human Basic fibroblast growth factor or functionally equivalent analog thereof in water or in a buffered aqueous solution containing EDTA; adjusting the pH of said solution to a value of about 8.0, treating said pH adjusted solution with the desired derivatizing agent; eliminating from the resulting mixture the derivatizing agent in excess by loading the said mixture on a column packed with a cationic exchange resin and extensively washing the column with a buffered solution, eluting the derivatized protein with a linear gradient of sodium chloride and desalting the derivatized protein by passing it through a Sephadex G-25 column.

7. A pharmaceutical composition comprising an effective amount of a derivatized recombinant human basic fibroblast growth factor or a functionally equivalent analog thereof according to any of claims 1 to 5 together with a pharmaceutically acceptable excipient.

8. A derivatized recombinant human basic fibroblast growth factor or a functionally equivalent analog thereof according to any of claims 1 to 5 for use in the preparation of a medicament that can be advantageously used for accelerating wound healing, revascularizing ischaemic tissues or regenerating damaged neural tissues.

9. The use of a derivatized recombinant human basic fibroblast growth factor or a functionally equivalent analog thereof according to any of claims 1 to 5 for use in the healing of wounds, ulcers or burns; regeneration of damaged neural tissues; aiding tissue transplant or healing bone graft; or revascularization of ischaemic tissues.

Fig. ¹ - Tryptic maps of bFGF (A) vs. CM-FGF (B)

Fig. 2 - V8 protease maps of PE-CM-FGF (A) vs.
CM-FGF (B)

Figure 3

## STABILITY AT 4°C

Figure 4

EP 0 494 664 A1

Figure 5

Proliferation assay on BAEC

Figure 6

EP 0 494 664 A1

Receptor binding assay on BHK cells

Figure 7

EP 0 494 664 A1

European Patent
Office

PARTIAL EUROPEAN SEARCH REPORT
which under Rule 45 of the European Patent Convention
shall be considered, for the purposes of subsequent
proceedings, as the European search report

Application Number

EP  92 10 0257

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 904 832  (AMGEN, INC.)<br>* Page 30, lines 18-29; page 32, lines 26-34; pages 48-55; claims *<br>--- | 1-9 | C 12 N   15/12<br>A 61 K   37/02<br>C 07 K    3/08<br>C 07 K   13/00 |
| X | JOURNAL OF BIOLOGICAL CHEMISTRY, vol. 263, no. 34, 5th December 1988, pages 18452-18458, Baltimore, US; G.M. FOX et al.: "Production, biological activity, and structure of recombinant basic fibroblast growth factor and an analog with cysteine replaced by serine"<br>* Whole article, and especially page 18453, right-hand column: "S-carboxymethylation of bFGF" *<br>--- | 1-4 | |
| Y | IDEM<br>--- | 1-6 | |
| Y | EP-A-0 355 460  (AMERICAN CYANAMID CO.)<br>* Page 2, line 31 - page 3, line 20; page 10, line 50 - page 11, line 4 *<br>---                              -/- | 1-6 | |

TECHNICAL FIELDS
SEARCHED (Int. Cl.5)

C 12 N
C 07 K
A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims
Claims searched completely :
Claims searched incompletely :
Claims not searched :
Reason for the limitation of the search:

Remark: Although claim 9
is  directed to a method of
treatment of the human/animal
body (Art. 52(4) EPC) the search
has been carried out and based on
the alleged effects of the
compound/composition

| Place of search | Date of completion of the search | Examiner |
|---|---|---|
| THE HAGUE | 16-03-1992 | ANDRES S.M. |

European Patent
Office

**PARTIAL EUROPEAN SEARCH REPORT**

Application Number

EP   92 10 0257

| | DOCUMENTS CONSIDERED TO BE RELEVANT | | CLASSIFICATION OF THE APPLICATION (Int. Cl.5) |
|---|---|---|---|
| Category | Citation of document with indication, where appropriate, of relevant passages | Relevant to claim | |
| A | JOURNAL OF BIOLOGICAL CHEMISTRY, vol. 265, no. 10, 5th April 1990, pages 5504-5511, Baltimore, US; D.N. BREMS et al.: "Equilibrium denaturation of human growth hormone and its cysteine-modified forms" * Whole document * | | |
| A | EP-A-0 290 029  (RORER INTERNATIONAL (OVERSEAS) INC.) * Page 12, "cycle 7"; page 13, line 45 - page 15, line 47 * | | |
| P,X | EP-A-0 458 064  (AMERICAN CYANAMID CO.) * Page 2, line 50 - page 3, line 43; claims 4,18-19 * | 1-6 | TECHNICAL FIELDS SEARCHED (Int. Cl.5) |